# FASCICULE DE BREVET EUROPEEN

(11) **EP 0 548 039 B1**
(45) Date de publication et mention de la délivrance du brevet: **06.08.1997**
(21) Numéro de dépôt: 92870196.0
(22) Date de dépôt: 15.12.1992
(51) Int. Cl.: A61F 13/20

(54) **Tampon périodique interne pour la protection féminine**
Menstruationstampon zum Schutz von Frauen
Catamenial tampon for feminine protection

(30) Priorité: 16.12.1991 BE 9101138
(43) Date de publication de la demande: 23.06.1993
(73) Titulaire: Legrand, Marie-Thérèse, B-4053 Embourg (BE)
(72) Inventeur: Legrand, Marie-Thérèse, B-4053 Embourg (BE)
(74) Mandataire: Vanderperre, Robert

(56) Documents cités:
- CH-A- 565 553
- US-A- 4 185 631
- US-A- 4 212 301

## Description

La présente invention concerne les tampons en matière absorbante destinés à absorber le flux sanguins périodique lors de la menstruation chez la femme.

On connaît par le brevet US-A-4.212.301 un tampon digital, à introduire dans la cavité vaginale, constitué d'un corps en matière fibreuse recouvert d'une feuille en matière perméable au fluide, ledit corps se présentant sous une forme allongée conique, dont les parois évasées seront comprimées par la pression vaginale pour former un long tampon absorbant.

Un tel tampon, s'il permet d'absorber le flux sanguin périodique, est de réalisation relativement compliquée et ne permet pas les relations sexuelles pendant les règles.

Par suite, il a paru avantageux de créer un tampon périodique de fabrication simple, de maniement aisé, d'une grande efficacité dans sa capacité d'absorption et, du fait qu'en place il n'obstrue pas le vagin, rendant possible une relation sexuelle.

Un tampon périodique suivant l'invention comprenant un petit coussin en matière absorbante de forme arrondie et recouvert d'une enveloppe perméable et un cordonnet de retrait fixé au coussin, est caractérisé en ce que le coussin a une base présentant un creux central préformé dimensionné pour couvrir le col de l'utérus de l'utilisatrice, et une face opposée bombée, ladite enveloppe étant un pansement s'étendant autour du coussin en formant un bord circulaire plat et le cordonnet de retrait s'étendant vers l'extérieur en passant entre le coussin et le bord circulaire plat afin de permettre l'extraction du tampon après usage.

Contrairement aux tampons périodiques internes connus qui s'introduisent dans le vagin par leur côté bombé, le tampon périodique suivant l'invention s'introduit par sa base présentant le creux central préformé de manière à venir couvrir le col de l'utérus de l'utilisatrice.

Le pansement est totalement sans couture et est recouvert d'un adhésif sur sa face interne.

Avantageusement, le pansement est constitué d'un sparadrap anallergique formé d'un non-tissé en polyester recouvert d'un film adhésif anallergique.

On peut encore citer comme avantages d'un tampon périodique suivant l'invention le fait que la menstruation peut survenir sans inconvénient pour la femme et le fait que son utilisation n'empêche pas les relations sexuelles.

Pour mieux faire comprendre l'invention celle-ci est décrite maintenant plus en détail sur la base du dessin annexé, à titre d'exemple uniquement, montrant en :

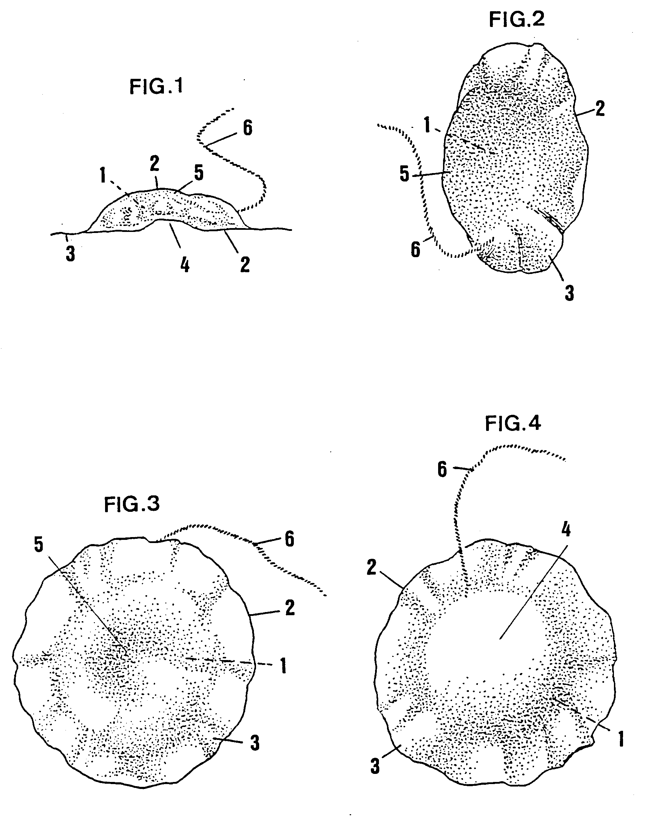

Figure 1 une vue de profil d'un tampon périodique suivant l'invention;
Figure 2 une vue en perspective du tampon de figure 1;
Figure 3 une vue de la face du tampon, côté base, et
Figure 4 une vue de l'autre face du tampon.

Le tampon périodique représenté au dessin et conforme à l'invention comporte une petit coussin d'ouate absorbante 1 de forme arrondie. Le diamètre de ce petit coussin est d'environ 4 cm.

Le petit coussin d'ouate 1 est recouvert sur ses deux faces d'un pansement doux, lisse et perméable 2. Lesdits pansements dépassent le coussin d'ouate tout autour pour former un bord circulaire plat 3 sur environ 1 cm, de telle manière que le diamètre du tampon est d'environ 6 cm.

La forme du tampon est celle d'un petit chapeau, la face constituant la base présentant un creux central 4 d'environ 2 cm et son autre face un bombage 5.

A l'intérieur du coussin d'ouate 1 est fixée l'extrémité d'un cordonnet de retrait 6, dont l'autre extrémité ressortant entre ledit coussin 1 et le bord circulaire plat 3 s'étend sur quelques centimètres, par exemple 6 cm.

On utilise avantageusement pour former le petit coussin une ouate cent pour cent coton, 680 mmg, conforme à la pharmacopée européenne.

Pour former le pansement 2 on utilise avantageusement un sparadrap anallergique, c'est-à-dire un non-tissé très doux, souple, poreux, aéré, cent pour cent polyester recouvert d'un film adhésif anallergique, contenant une colle à base d'eau assurant un haut degré d'hypoallergie. Le film adhésif et le non-tissé sont perforés régulièrement sur toute leur superficie avec vingt-cinq perforations au centimètre carré. Egalement, on utilise avantageusement du coton cent pour cent pour le cordonnet rond 6.

Un tampon suivant l'invention s'adapte parfaitement : en effet son creux préformé à la base permet une adhérence totale au col de l'utérus tandis que son autre face bombée réalise une absorption maximale.

Un tel tampon s'adapte impeccablement à la morphologie féminine pour absorber le flux sanguin au niveau du col de l'utérus sans le moindre écoulement de sang dans le vagin.

Grâce à sa matière de recouvrement le tampon ne peut se dilater dans le vagin.

## Revendications

1. Tampon périodique interne pour la protection féminine, comprenant un petit coussin (1) en matière absorbante de forme arrondie et recouvert d'une enveloppe perméable (2), et un cordonnet de retrait (6) fixé au coussin, caractérisé en ce que le coussin (1) a une base présentant un creux central (4) préformé dimensionné pour couvrir le col de l'utérus de l'utilisatrice, et une face opposée bombée, ladite enveloppe étant un pansement s'étendant autour du coussin en formant un bord circulaire plat (3) et le cordonnet de retrait (6) s'étendant vers l'extérieur en passant entre le coussin (1) et le bord circulaire plat (3) afin de permettre l'extraction du tampon après usage.

2. Tampon périodique suivant la revendication 1, caractérisé en ce que le pansement est totalement sans couture et en ce que le pansement (2) est recouvert d'un adhésif sur sa face interne.

3. Tampon périodique suivant la revendication 1 ou 2, caractérisé en ce que le coussin (1) est cent pour cent coton.

4. Tampon périodique suivant l'une quelconque des revendications 1 à 3, caractérisé en ce que le pansement est un non-tissé.

5. Tampon périodique suivant l'une quelconque des revendications 1 à 3, caractérisé en ce que le pansement est constitué d'un sparadrap anallergique formé d'un non-tissé en polyester recouvert d'un film adhésif anallergique.

6. Tampon périodique suivant l'une quelconque des revendications 1 à 5, caractérisé en ce que le cordonnet (6) est cent pour cent coton.

## Claims

1. Internal menstrual vagina tampon for female protection, comprising a small pouch (1) made of absorbent material of rounded form and sheated with a permeable envelope (2) and a withdrawal cord (6) fixed to the pouch, characterised in that the pouch (1) has a base with a preformed central hollow (4) sized so as to cover the cervix of the uterus of the user, and an opposite convex face, said envelope being constituted of a dressing extending around the pouch so forming a flat circular rim (3) and the withdrawal cord (6) exiting from between the pouch (1) and the flat circular rim (3) thereby to permit the tampon to be extracted after use.

2. Menstrual tampon according to claim 1, characterised in that the dressing is totally free of stitching and in that it is coated with an adhesive on the internal face thereof.

3. Menstrual tampon according to claim 1 or 2, characterised in that the pouch (1) is of a one hundred percent cotton.

4. Menstrual tampon according to either of claims 1 to 3, characterised in that the dressing is constituted of an unwoven fabric.

5. Menstrual tampon according to either of claims 1 to 3, characterised in that the dressing is constituted of a hypo-allergic type plaster, formed of an unwoven polyester fabric coated with a hypo-allergic adhesive film.

6. Menstrual tampon according to either of claims 1 to 5, characterised in that the withdrawal cord (6) is of one hundred percent cotton.

## Patentansprüche

1. Intravaginaler Tampon zum Menstruationsschutz der Frau, der ein kleines Polster (1) aus einem absorbierenden Material von runder Form, das mit einer permeablen Umhüllung (2) versehen ist, und ein an dem Polster befestigtes Rückholbändchen (6) umfaßt,
**dadurch gekennzeichnet, daß**
das Polster (1) eine Grundflache mit einer vorgeformten zentralen Mulde (4), die so dimensioniert ist, daß der Gebärmutterhals der Anwenderin bedeckt ist, und eine gegenüberliegende gewölbte Seite aufweist, wobei die Umhüllung aus einer Hülle besteht, die sich um den Saugkörper herum erstreckt, wodurch ein kreisförmiger flacher Rand (3) gebildet wird, und wobei das Rückholbandchen (6) sich zwischen dem Saugkörper (1) und dem flachen kreisförmigen Rand (3) hindurch nach außen erstreckt, um das Entfernen des Tampons nach der Verwendung zu gewährleisten.

2. Tampon nach Anspruch 1, dadurch gekennzeichnet, daß die Hülle vollkommen nahtlos ist und dadurch, daß die Hülle (2) an ihrer inneren Fläche mit einem Kleber bedeckt ist.

3. Tampon nach Anspruch 1 oder 2, dadurch gekennzeichnet, daß das Polster (1) aus 100 % Baumwolle besteht.

4. Tampon nach einem der Ansprüche 1 bis 3, dadurch gekennzeichnet, daß die Hülle aus einem nicht gewebten Material besteht.

5. Tampon nach einem der Ansprüche 1 bis 3, dadurch gekennzeichnet, daß die Hülle aus einem keine Allergie hervorrufenden Pflaster besteht, das aus einem ungewebten Material aus Polyester gebildet wird und mit einem keine Allergie hervorrufenden Haftfilm bedeckt ist.

6. Tampon nach einem der Ansprüche 1 bis 5, dadurch gekennzeichnet, daß das Bändchen (6) aus 100 % Baumwolle besteht.
